(19) Europäisches Patentamt
European Patent Office
Office européen des brevets

(11) Numéro de publication: **0 187 056**
**A1**

(12) **DEMANDE DE BREVET EUROPEEN**

(21) Numéro de dépôt: **85402144.1**

(22) Date de dépôt: **06.11.85**

(51) Int. Cl.⁴: **C 09 K 19/30**, C 07 C 121/75

(30) Priorité: **09.11.84 FR 8417111**

(43) Date de publication de la demande: **09.07.86**
**Bulletin 86/28**

(84) Etats contractants désignés: **BE CH DE GB LI NL**

(71) Demandeur: **THOMSON-CSF, 173, Boulevard Haussmann, F-75379 Paris Cedex 08 (FR)**

(72) Inventeur: **Barny, Pierre, THOMSON-CSF SCPI 173, bld Haussmann, F-75379 Paris Cedex 08 (FR)**
Inventeur: **Dubois, Jean-Claude, THOMSON-CSF SCPI 173, bld Haussmann, F-75379 Paris Cedex 08 (FR)**
Inventeur: **Ravaux, Gilles, THOMSON-CSF SCPI 173, bld Haussmann, F-75379 Paris Cedex 08 (FR)**

(74) Mandataire: **Lepercque, Jean et al, THOMSON-CSF SCPI 19, avenue de Messine, F-75008 Paris (FR)**

(54) **Composé organique présentant une phase smectique A, mélange comprenant ce composé, et procédé de fabrication.**

(57) L'invention se rapporte à une famille de composés organiques du type ester de 1(4 hydroxyphényl) 2(4 cyanophényl) éthane qui présente une phase mésomorphe de type smectique A, aux mélanges obtenus à partir de ces composés ainsi qu'au procédé de fabrication des molécules de cette famille.

Le composé selon l'invention répond à la formule chimique générale:

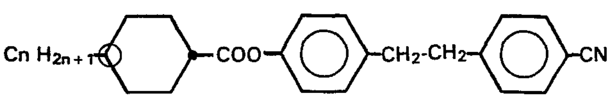

n pouvant être compris entre 1 et 15.

L'invention s'applique au domaine des cristaux liquides et aux dispositifs de visualisation utilisant de tels cristaux.

EP 0 187 056 A1

# COMPOSE ORGANIQUE PRESENTANT UNE PHASE SMECTIQUE A, MELANGE COMPRENANT CE COMPOSE ET PROCEDE DE FABRICATION

L'invention concerne une famille de composés organiques du type ester de 1-(4 hydroxyphényl) 2-(4' cyanophényl) éthane qui présente une mésophase de type smectique A et éventuellement une mésophase nématique. L'invention concerne également le procédé de fabrication des molécules de cette famille, ainsi que les mélanges obtenus à partir de ces esters et possédant également une phase smectique A.

Il existe dans l'art connu toute une gamme de cristaux liquides présentant une phase smectique A. Ceux-ci présentent généralement une plage de mésomorphie assez restreinte. Afin de pallier ces inconvénients, l'invention propose des composés organiques permettant l'obtention de plages beaucoup plus importantes.

Les composés selon l'invention possèdent la propriété, lorsqu'on les mélange à des cristaux liquides smectiques A, d'élargir la plage smectique que présentaient ces produits. Ceci est un avantage lorsque de tels mélanges sont utilisés dans des dispositifs de visualisation qui voient leur gamme d'utilisation s'élargir à la fois vers les hautes et vers les basses températures.

Les composés selon l'invention répondent à la formule chimique générale :

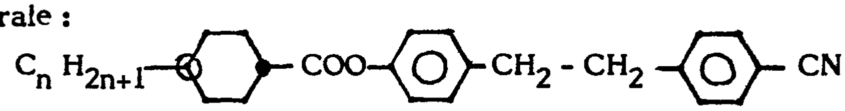

avec $1 \leqslant n \leqslant 15$.

Le symbole 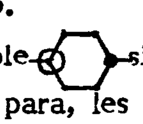 signifie que l'on a affaire à un dérivé du cyclohexane disubstitué en para, les substituants étant en position équatoriale ce qui conduit à des dérivés trans.

Les composés répondant à cette formule peuvent être définis par la dénomination suivante : 1-(4 alkyl cyclohexyl carbonyloxyphényl) 2-(4'cyanophényl) éthane.

L'invention a donc pour objet un composé organique présentant au moins une phase mésomorphe de type smectique A, caractérisé en ce qu'il

répond à la formule chimique générale :

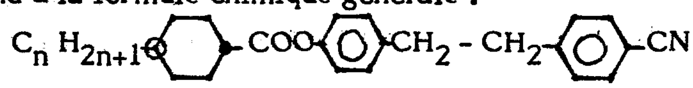

pour laquelle $1 \leqslant n \leqslant 15$.

L'invention a aussi pour objet un mélange de cristaux liquides présentant au moins une phase smectique A, caractérisé en ce qu'il comporte au moins l'un des composés organiques cités ci-dessus.

L'invention a encore pour objet un procédé de fabrication d'un composé tel que cité ci-dessus, caractérisé en ce que ledit composé est un produit de la réaction du chlorure d'acide 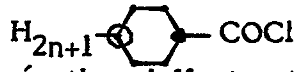 et du phénol 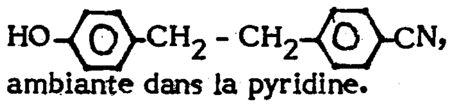, ladite réaction s'effectuant à température ambiante dans la pyridine.

L'invention sera mieux comprise et d'autres avantages apparaîtront au moyen de la description qui va suivre et de la figure annexée qui est un diagramme de phase isobare.

La description qui va suivre portera sur le procédé général de synthèse des molécules selon l'invention, ainsi que sur les propriétés mésomorphes des cristaux liquides correspondants. De plus, un exemple d'incorporation d'un composé selon l'invention dans un matériau smectique A destiné à un affichage utilisant un effet mixte thermique et électrique, mettra en évidence l'élargissement de la gamme d'utilisation de cet affichage vers les basses et les hautes températures.

PROCEDE GENERAL DE SYNTHESE

Les composés organiques selon l'invention peuvent être obtenus en 9 étapes pour $n \geqslant 9$ à partir des produits commerciaux suivants : l'acide 4 bromo-phénylacétique, le méthoxybenzène et l'alkyl benzène ou en 7 étapes pour $n \leqslant 8$ car dans ce cas les acides alkylbenzoïques sont disponibles dans le commerce.

Réaction 1 : Obtention du chlorure de 4 bromophénylacétyle. Ce chlorure d'acide est obtenu par action du chlorure de thionyle sur l'acide 4 bromophénylacétique.

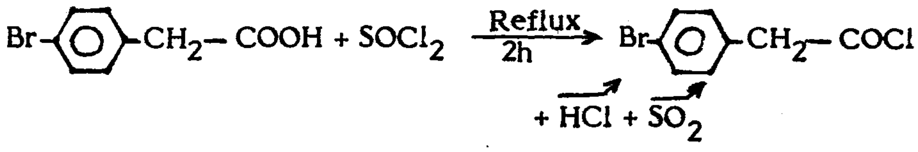


Réaction 2 : Synthèse du 4-(4 bromophénylacétyl) 4'-méthoxyphényle. Le 4-(4 bromophénylacétyl) 4'méthoxyphényle est obtenu par action du chlorure de 4 bromophénylacétyle sur le méthoxybenzène en présence de chlorure d'aluminium et de chlorure de méthylène, à une température de 5° C. (Réaction de FRIEDEL-CRAFT).

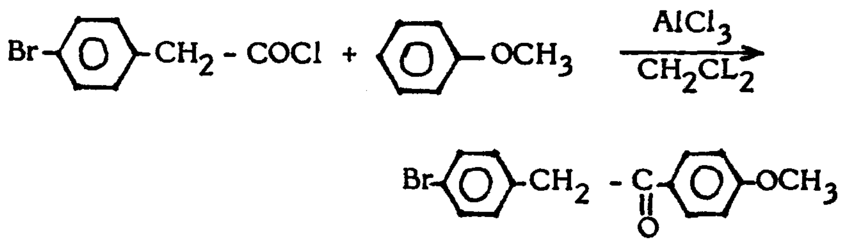


Réaction 3 : Obtention du 1-(4 bromophényl) 2-(4'hydroxyphényl) éthane.

Le 4-(4 bromophénylacétyl) 4'-méthoxyphényle est réduit en 1-(4 bromophényl) 2-(4'hydroxyphényl) éthane selon la réaction de WOLF-KISHNER modifiée par HUANG-MINLON, par action de l'hydrazine en milieu basique et en présence de diéthylène glycol. Le milieu basique est obtenu par de la potasse. Cette réaction s'accompagne d'une coupure totale de la fonction éther.

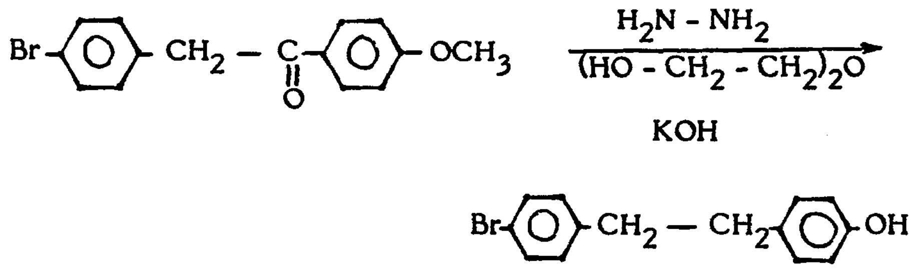


Le phénol ainsi obtenu est purifié en le transformant en acétate au moyen du chlorure d'acétyle en présence de pyridine. L'acétate est chromatographié sur de la silice avec le toluène comme éluant, puis saponifié en milieu potasse alcoolique. Ce qui conduit au phénate de potassium que l'on transforme en phénol par action de l'acide

chlorhydrique.

Réaction 4 : Synthèse du 1-(4 cyanophényl) 2-(4'hydroxyphényl) éthane.

Le 1-(4 cyanophényl) 2-(4'hydroxyphényl) éthane est obtenu par cyanuration du 1-(4 bromophényl) 2-(4' hydroxyphényl) éthane au moyen du cyanure cuivreux en utilisant la N méthyl pyrrolidone comme solvant.

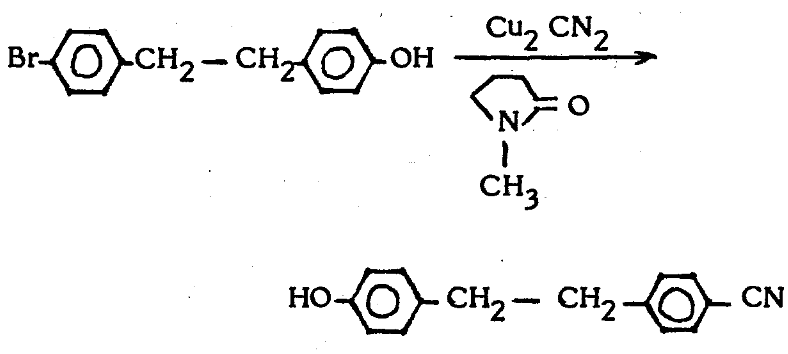


Réaction 5 : Obtention du 4 alkyl 4'acétyl phényle.

Le 4 alkyl 4'acétyl phényle est obtenu par action du chlorure d'acétyle sur l'alkyl benzène en présence de chlorure d'aluminium et du tétrachlorure de carbone à 5° C (Réaction de FRIEDEL-CRAFT).

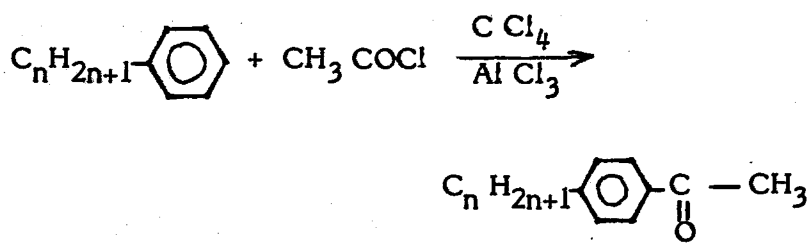


Réaction 6 : Synthèse des acides 4 alkyl benzoïques.

Les acides 4 alkyl benzoïques sont obtenus par oxydation des 4 alkyl 4' acétyl phényles au moyen de l'hypobromite de sodium. Le sel de sodium résultant est acidifié par l'acide chlorhydrique.

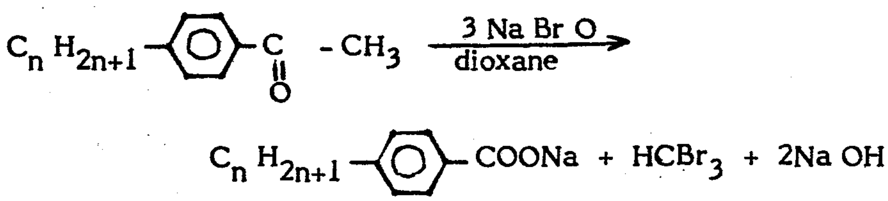

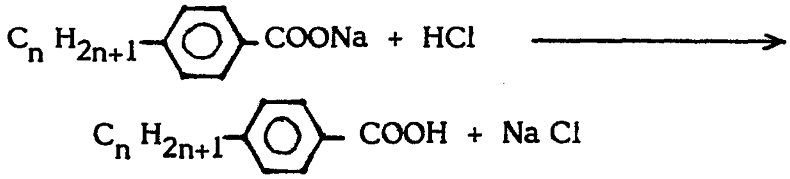

<u>Réaction 7</u> : Obtention de l'acide trans 4 alkyl cyclohexane carboxylique.

Par hydrogénation à 200° C, sous un pression de 200 bars en présence de Nickel de Raney W2 et en milieu basique, des acides 4 alkyl benzoïques, on obtient un mélange cis-trans d'acide 4 alkyl cyclohexane carboxylique riche en isomère trans.

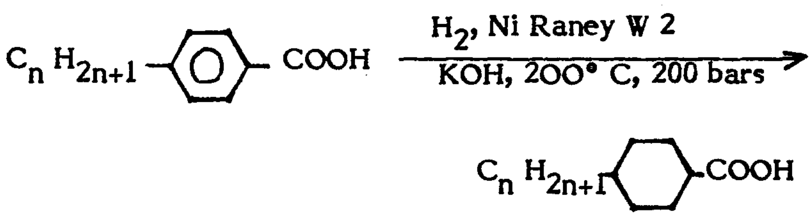


L'isomère trans est séparé du mélange par formation d'un composé d'inclusion avec la thiourée.

<u>Réaction 8</u> : Synthèse des chlorures de trans 4 alkyl cyclohexane carbonyloxy.

Les chlorures d'acide sont obtenus par action du chlorure de thionyle sur les acides trans 4 alkyl cyclohexane carboxyliques.

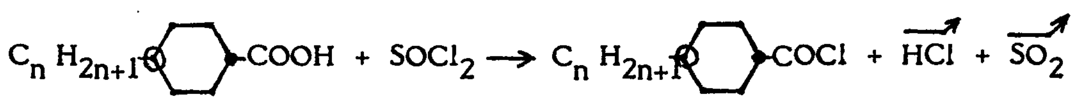

<u>Réaction 9</u> : Synthèse des 1-(4 alkylcyclohexylcarbonyloxyphényl)2-(4' cyanophényl) éthanes.

La synthèse de ces esters se fait à partir du chlorure d'acide 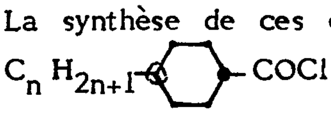 et du phénol

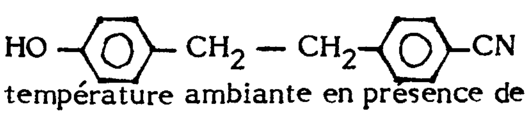 obtenu par la réaction 4, à température ambiante en présence de pyridine.

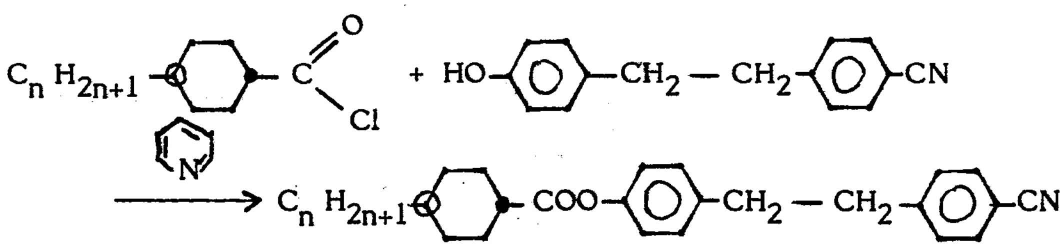

Le mode opératoire qui vient d'être décrit est valable quelles que soient les valeurs de n pourvu que l'on respecte les proportions molaires.

Les composés selon l'invention répondent aux dénominations suivantes :

1- (4 méthyl cyclohexyl carbonyloxyphényl) 2 - (4' cyanophényl) éthane.

1 - (4 éthyl cyclohexyl carbonyloxyphényl) 2 - (4' cyanophényl) éthane.

1 - (4 propyl cyclohexyl carbonyloxyphényl) 2 - (4' cyanophényl) éthane.

1 - (4 butyl cyclohexyl carbonyloxyphényl) 2 - (4' cyanophényl) éthane.

1 - (4 pentyl cyclohexyl carbonyloxyphényl) 2 - (4' cyanophényl) éthane.

1 - (4 hexyl cyclohexyl carbonyloxyphényl) 2 - (4' cyanophényl) éthane.

1 - (4 heptyl cyclohexyl carbonyloxyphényl) 2 - (4' cyanophényl) éthane.

1 - (4 octyl cyclohexyl carbonyloxyphényl) 2 - (4' cyanophényl) éthane.

1 - (4 nonyl cyclohexyl carbonyloxyphényl) 2 - (4' cyanophényl) éthane.

1 - (4 décyl cyclohexyl carbonyloxyphényl) 2 - (4' cyanophényl) éthane.

1 - (4 undécyl cyclohexyl carbonyloxyphényl) 2 - (4' cyanophényl) éthane.

1 - (4 dodécyl cyclohexyl carbonyloxyphényl) 2 - (4' cyanophényl) éthane.

1 - (4 tridécyl cyclohexyl carbonyloxyphényl) 2 - (4' cyanophényl) éthane.

1 - (4 tétradécyl cyclohexyl carbonyloxyphényl) 2 - (4' cyanophényl) éthane.

et 1 - (4 pentadécyl cyclohexyl carbonyloxyphényl) 2 - (4' cyanophényl) éthane.

PROPRIETES DES CORPS SYNTHETISES

A titre d'exemple non limitatif, l'analyse calorimétrique associée à la méthode de contact a permis de déterminer les caractéristiques suivantes pour l'ester selon l'invention correspondant à n = 9. Cet ester possède les transitions de phase suivantes :

K 117,4°C [5,5] $S_A$ 147,6° [0,14] N 150,3° [0,8] I.

Les lettres K, $S_A$, N et I désignent respectivement les phases cristalline, smectique A, nématique et isotrope. Les valeurs entre crochets sont les enthalpies de transition exprimées en kcal/mole.

La nature des mésophases a été déterminée par l'étude de l'isomorphisme au microscope optique des composés étudiés avec des substances connues. A titre d'exemple, la figure annexée représente un diagramme de phase isobare obtenu par la mise en contact du 1 (4 nonyl cyclohexyl carbonyloxyphényl) 2-(4'cyanophényl) éthane (ou composé A), selon l'invention avec un produit du type ester de 2 hydroxyfluorène substitué de formule :

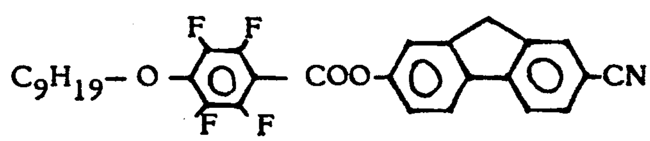

(ou composé B). Le composé B possède la succession de phases suivantes :

K 109°C $S_A$ 140,5°C N 152,5°C I

Cet ester est décrit dans la demande de brevet français de la Demanderesse publiée sous le numéro FR-A-2.538.804. Sur le diagramme, l'axe des ordonnées de gauche correspond à 100 % de composé A tandis que celui de droite correspond à 100 % de composé B. Les axes des ordonnées sont gradués en degrés Celsius, l'axe des abscisses en pourcentage de composé B dans le mélange.

Quelles que soient les proportions des composés A et B dans le mélange, celui-ci présente toujours une succession de phases : cristalline - smectique A - nématique - isotrope.

Il entre dans le cadre de l'invention d'utiliser les composés organiques selon l'invention comme cristaux liquides présentant une phase smectique A, seuls, en mélange entre eux ou avec d'autres cristaux liquides smectiques A, afin d'en élargir le domaine d'utilisation en température. A ce titre, ils peuvent être avantageusement utilisés dans des dispositifs de visualisation.

L'exemple qui va suivre va porter sur l'influence de l'introduction d'un composé selon l'invention dans un mélange de deux cristaux liquides appelés composés C et D. Le composé C est le 4 octyl 4' cyanobiphényle de formule $C_8H_{17}$ 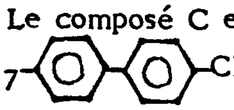 CN qui possède les transitions de phase suivantes :
K21,5° C $S_A$ 33,5° N 40,5° C I. Le composé D est le 4 décyl 4' cyanobiphényle de formule $C_{10}$ $H_{21}$ 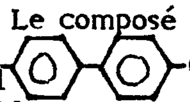 CN qui possède les transitions de phases suivantes : K 43,5°C $S_A$ 51°C I. L'eutectique formé à partir des composés C et D possède un point de fusion de 13°C, une transition smectique A- nématique à 38,1°C et une température de clarification de 43,3°C donc une plage de mésomorphie de 30,3°C.

L'introduction du 1-(4 nonyl cyclohexylcarbonyloxyphényl) 2-(4'cyanophényl) éthane conduit à un eutectique ternaire comprenant 63,2 % de composé C, 21,4 % de composé D et 15,4 % d'ester selon l'invention. Cet eutectique possède un point de fusion de 8,9° C, une température de transition smectique A - nématique de 63,4° C et une température de clarification de 68,6° C. La plage mésomorphe de ce mélange ternaire s'étend donc sur 59,7° C. Par rapport au mélange binaire décrit précédemment on constate que la plage mésomorphe est plus étendue et que les températures limites de cette plage sont bien repoussées vers les basses et les hautes températures.

## REVENDICATIONS

1. Composé organique présentant au moins une phase mésomorphe de type smectique A, caractérisé en ce qu'il répond à la formule chimique générale :

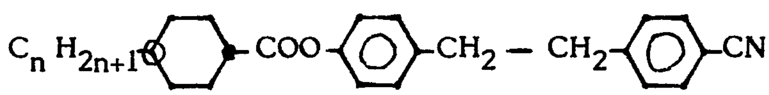

pour laquelle $1 \leqslant n \leqslant 15$.

2. Composé organique selon la revendication 1, caractérisé en ce que : n = 9.

3. Mélange de cristaux liquides présentant au moins une phase smectique A, caractérisé en ce qu'il comporte au moins un composé organique selon l'une des revendications 1 ou 2.

4. Mélange selon la revendication 3, caractérisé en ce qu'il comprend 63,2 % de 4 octyl 4' cyano biphényle, 21,4 % de 4 décyl 4'cyano biphényle et 15,4 % dudit composé organique pour lequel n = 9.

5. Procédé de fabrication d'un composé organique selon l'une des revendications 1 ou 2, caractérisé en ce que ledit composé est un produit de la réaction du chlorure d'acide 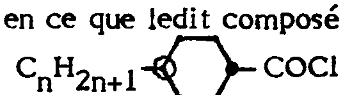 et du phénol 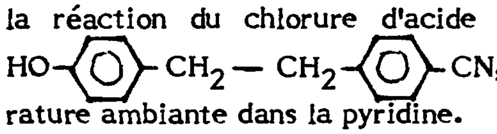, ladite réaction s'effectuant à température ambiante dans la pyridine.

6. Procédé de fabrication selon la revendication 5, caractérisé en ce qu'il comprend également les étapes suivantes :

a) obtention du chlorure de 4 bromophénylacétyle par action du chlorure de thionyle sur l'acide 4 bromophénylacétique ;

b) synthèse du 4-(4 bromophénylacétyle) 4'-méthoxyphényle par la réaction de FRIEDEL-CRAFT ;

c) réduction du produit obtenu à l'étape précédente par une réaction de WOLF-KISHNER, cette réaction s'accompagnant de la déméthylation du phénol.

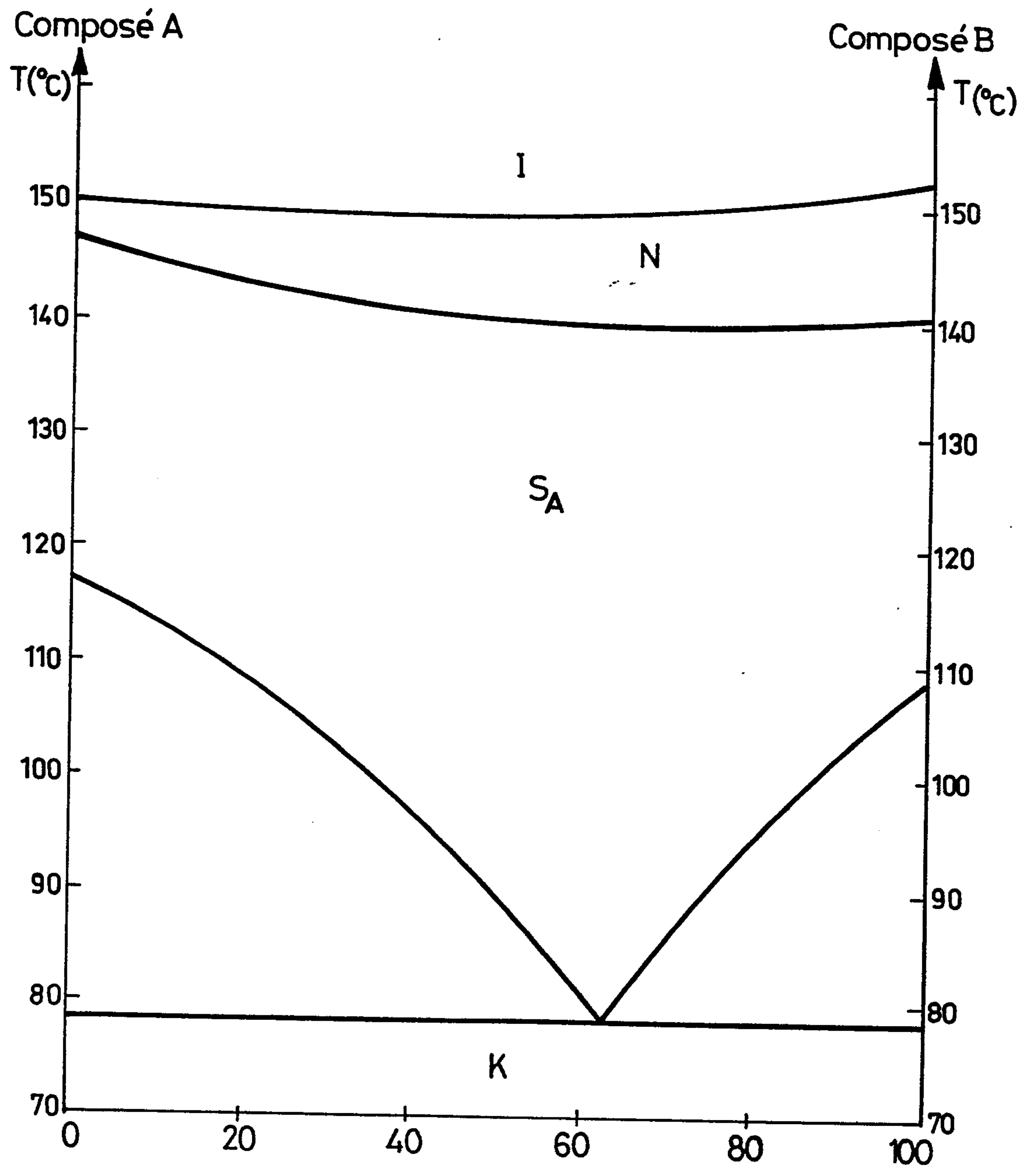
Composé A
T(°C)
Composé B
T(°C)
I
N
$S_A$
K
150
140
130
120
110
100
90
80
70
0
20
40
60
80
100

0187056

Office européen des brevets

# RAPPORT DE RECHERCHE EUROPEENNE

Numero de la demande

EP 85 40 2144

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | DE-A-3 339 216 (DAINIPPON) * Revendications; page 2, lignes 5-18,15-21; exemples 7-10 * --- | 1 | C 09 K 19/30<br>C 07 C 121/75 |
| A | GB-A-2 123 409 (DAINIPPON) * Revendications 1-13 * ----- | 1 | |

| DOMAINES TECHNIQUES RECHERCHES (Int. Cl.4) |
|---|
| C 09 K<br>C 07 C |

Le présent rapport de recherche a été établi pour toutes les revendications

| Lieu de la recherche | Date d'achèvement de la recherche | Examinateur |
|---|---|---|
| LA HAYE | 14-02-1986 | BOULON A.F.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82